# EUROPEAN PATENT APPLICATION

(11) **EP 4 670 660 A1**
(43) Date of publication of application: **31.12.2025**
(21) Application number: 23933144.0
(22) Date of filing: 09.05.2023
(51) Int. Cl.: A61B 18/14, A61N 1/36, A61N 1/05, A61B 34/30, A61B 18/00, A61B 17/00

(54) **ELECTRODE APPARATUS FOR BLOCKING OR CONTROLLING NERVES INSIDE BODY AND SURGICAL ROBOT USING SAME**

(30) Priority: 14.04.2023 KR 20230049322
(71) Applicant: Deepqure Inc., Seoul 06243 (KR)
(72) Inventor: BACH, Du Jin, Seoul 06243 (KR); JO, Seok Hyeon, Seoul 06243 (KR); YOON, Young Il, Seoul 06243 (KR); KIM, Kee Wan, Seoul 06243 (KR); PARK, Chan Ho, Seoul 06243 (KR); PARK, Jong Sun, Seoul 06243 (KR)
(74) Representative: Manitz Finsterwald Patent- und Rechtsanwaltspartnerschaft mbB
(86) International application number: PCT/KR2023/006267
(87) International publication number: WO 2024/214856

(57) **Abstract**

An electrode apparatus for blocking or controlling nerves in a body according to an embodiment of the present disclosure includes: a main body including a shaft; an electrode unit formed to be drawn out from one end of the shaft and configured to denervate or modulate at least a part of nerves on a tube in the body; an electrode guide coupled to at least a part of the electrode unit and to guide the electrode unit to be brought into contact with the tube in the body; an electrode driving unit including a tensile force maintenance unit connected to the electrode unit and configured to move the electrode unit in forward and backward directions and provide a tensile force to the electrode unit; an electrode guide driving unit connected to the electrode guide and configured to move the electrode guide in the forward and backward directions; and a motor interlocking unit connected to the electrode driving unit and the electrode guide driving unit and configured to transfer a rotation amount generated by a plurality of motors to the electrode driving unit and the electrode guide driving unit.

A surgical robot according to another embodiment of the present disclosure includes: an electrode apparatus; a robot arm including a plurality of motors connected to the motor interlocking unit and configured to transfer a rotation amount to the electrode driving unit and the electrode guide driving unit through the motor interlocking unit; and a main body configured to support the robot arm.

## Description

### TECHNICAL FIELD

The present disclosure relates to an electrode apparatus for blocking or controlling nerves in body and a surgical robot using the same.

### BACKGROUND

Denervation is a surgical procedure intended to control an abnormally overactive autonomic nervous system by damaging specific nerves. For example, renal denervation can treat hypertension and heart diseases by damaging renal sympathetic nerves directed to the kidney, and pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves.

For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode. In particular, it is important to safely and accurately attach the electrode-formed component to the outer wall of the tube in the body so as not to damage the tube in the body, which can be easily damaged by external stimuli.

(Patent Document 1) Korean Patent Laid-open Publication No. 2013-0108401 (published on October 2, 2013)

### DISCLOSURE OF THE INVENTION

### PROBLEMS TO BE SOLVED BY THE INVENTION

The present disclosure is conceived to provide an electrode apparatus having a component that guides an electrode to enclose the circumference of a tube in a body.

Also, the present disclosure is conceived to provide an electrode apparatus configured to accurately bring a component including an electrode into close contact with an outer wall of the tube in the body without damaging the tube which can be easily damaged by external stimuli and thus to maintain a tensile force.

Further, the present disclosure is conceived to provide an electrode apparatus capable of finely regulating a tensile force of a component including an electrode when the component is brought into close contact with the outer wall of the tube in the body.

Furthermore, the present disclosure is conceived to provide a surgical robot in which an electrode apparatus according to an embodiment of the present disclosure can be used by being coupled to a robot arm including a plurality of motors.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

According to an aspect of the present disclosure, an electrode apparatus for blocking or controlling nerves in a body includes: a main body including a shaft; an electrode unit formed to be drawn out from one end of the shaft and configured to denervate or modulate at least a part of nerves on a tube in a body; an electrode guide coupled to at least a part of the electrode unit and to guide the electrode unit to be brought into contact with the tube in the body; an electrode driving unit including a tensile force maintenance unit connected to the electrode unit and configured to move the electrode unit in forward and backward directions and provide a tensile force to the electrode unit; an electrode guide driving unit connected to the electrode guide and configured to move the electrode guide in the forward and backward directions; and a motor interlocking unit connected to the electrode driving unit and the electrode guide driving unit and configured to transfer a rotation amount generated by a plurality of motors to the electrode driving unit and the electrode guide driving unit.

The tensile force maintenance unit may include: an electrode connection unit connected to one end of the electrode unit; an electrode tensile force regulation block configured to move in the forward and backward directions through a first rotation shaft coupled to the motor interlocking unit; and a spring configured to connect the electrode connection unit to the electrode tensile force regulation block and provide a tensile force to the electrode unit.

The electrode guide may include a plurality of joint units and a wire connecting the plurality of joint units to each other, and the electrode guide driving unit may include: a load block configured to move in the forward and backward directions through a second rotation shaft connected to the joint units and coupled to the motor interlocking unit; and a wire block configured to move in the forward and backward directions through a third rotation shaft connected to the wire and coupled to the motor interlocking unit.

When the electrode guide driving unit moves in the forward and backward directions, a first rotation speed of the second rotation shaft may be set to be higher than a second rotation speed of the third rotation shaft and the load block may move further in the forward and backward directions than the wire block.

When the electrode guide driving unit moves in the forward and backward directions, the electrode tensile force regulation block may move together in the forward and backward directions.

A third rotation speed of the first rotation shaft may be set to be lower than a first rotation speed of the second rotation shaft.

After the electrode guide driving unit moves in the forward direction and stops, the electrode tensile force regulation block may move in the backward direction.

After a predetermined tensile force is generated in the electrode unit as the electrode tensile force regulation block moves in the backward direction, the electrode tensile force regulation block may stop.

According to another aspect of the present disclosure, a surgical robot using an electrode apparatus includes: the electrode apparatus of Claim 1; a robot arm including a plurality of motors connected to the motor interlocking unit and configured to transfer a rotation amount to the electrode driving unit and the electrode guide driving unit through the motor interlocking unit; and a main body configured to support the robot arm.

The plurality of motors may include: a first motor configured to generate a rotation amount in order for the electrode tensile force regulation block to move in the forward and backward directions; a second motor configured to generate a rotation amount in order for the load block to move in the forward and backward directions; and a third motor configured to generate a rotation amount in order for the wire block to move in the forward and backward directions.

### EFFECTS OF THE INVENTION

In an electrode apparatus according to an embodiment of the present disclosure, an electrode guide is located to be in close contact with a tube in a body and gradually brings an electrode unit into close contact with an outer wall of the tube. Thus, it is possible to safely bring a component including an electrode into close contact with the outer wall of the tube without damaging the tube in the body which can be easily damaged by external stimuli.

Also, in the electrode apparatus, a motor interlocking unit transfers a rotation amount to each of an electrode driving unit and an electrode guide driving unit through a plurality of motors. Thus, it is possible to regulate the electrode unit to operate precisely when the electrode unit is brought into close contact with the outer wall of the tube.

In a surgical robot according to another embodiment of the present disclosure, an electrode apparatus can be used by being coupled to a robot arm including a plurality of motors configured to transfer a rotation amount through a motor interlocking unit.

The effects of the present disclosure are not limited to the aforementioned effects, and other effects, which are not mentioned above, will be apparent to a person with ordinary skill in the art from the following description.

### BRIEF DESCRIPTION OF THE DRAWINGS

**FIG. 1** is a perspective view of an electrode apparatus according to an embodiment of the present disclosure.
**FIG. 2** is an inner perspective view of the electrode apparatus according to an embodiment of the present disclosure.
**FIG. 3** illustrates a state where an electrode guide is located to guide an electrode unit to enclose a tube.
**FIG. 4** illustrates inner components of the electrode apparatus according to an embodiment of the present disclosure.
**FIG. 5** illustrates a motor interlocking unit of the electrode apparatus according to an embodiment of the present disclosure.
**FIG. 6A** illustrates an operation process of the electrode guide according to an embodiment of the present disclosure.
**FIG. 6B** illustrates an operation process of the electrode guide according to an embodiment of the present disclosure.
**FIG. 6C** illustrates an operation process of the electrode guide according to an embodiment of the present disclosure.
**FIG. 6D** illustrates an operation process of the electrode guide according to an embodiment of the present disclosure.
**FIG. 6E** illustrates an operation process of the electrode guide according to an embodiment of the present disclosure.
**FIG. 6F** illustrates an operation process of the electrode guide according to an embodiment of the present disclosure.
**FIG. 6G** illustrates an operation process of the electrode guide according to an embodiment of the present disclosure.
**FIG. 7A** illustrates an operation process of an electrode driving unit and an electrode guide driving unit according to an embodiment of the present disclosure.
**FIG. 7B** illustrates an operation process of the electrode driving unit and the electrode guide driving unit according to an embodiment of the present disclosure.
**FIG. 7C** illustrates an operation process of the electrode driving unit and the electrode guide driving unit according to an embodiment of the present disclosure.
**FIG. 7D** illustrates an operation process of the electrode driving unit and the electrode guide driving unit according to an embodiment of the present disclosure.
**FIG. 7E** illustrates an operation process of the electrode driving unit and the electrode guide driving unit according to an embodiment of the present disclosure.
**FIG. 7F** illustrates an operation process of the electrode driving unit and the electrode guide driving unit according to an embodiment of the present disclosure.
**FIG. 7G** illustrates an operation process of the electrode driving unit and the electrode guide driving unit according to an embodiment of the present disclosure.
**FIG. 8** is a perspective view illustrating a portion of joint units of the electrode apparatus according to an embodiment of the present disclosure.
**FIG. 9** is a perspective view of a surgical robot using the electrode apparatus according to another embodiment of the present disclosure.
**FIG. 10** illustrates the electrode apparatus coupled to a robot arm of the surgical robot according to another embodiment of the present disclosure.

### BEST MODE FOR CARRYING OUT THE INVENTION

Hereinafter, embodiments of the present disclosure will be described in detail with reference to the accompanying drawings to be readily implemented by a person with ordinary skill in the art to which the present invention belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Through the whole document, the term "comprises or includes" and/or "comprising or including" used in the document means that one or more other components, steps, operation and/or existence or addition of elements are not excluded in addition to the described components, steps, operation and/or elements unless context dictates otherwise. Also, through the whole document, the term "connected to" may be used to designate a connection or coupling of one element to another element and includes both an element being directly connected to another element, an element being connected to another element via another element, and an element being electronically connected to another element via another element. Further, through the whole document, the term "on" that is used to designate a position of one element with respect to another element includes both a case that the one element is adjacent to the other element and a case that any other element exists between these two elements. Furthermore, the expression "a first", "a second", "the first", or "the second" used in the present disclosure may modify various components regardless of the order and/or the importance, and is used only to distinguish one element from another element, but does not limit the corresponding elements. For example, a first direction and a second direction may indicate the same direction or may indicate different directions.

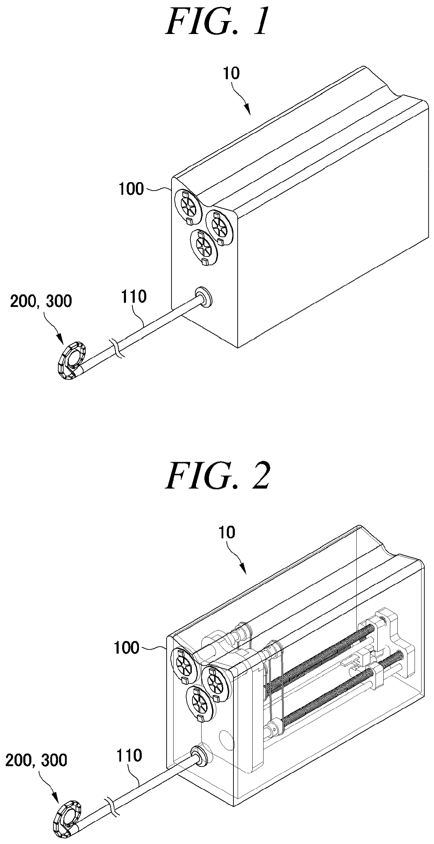

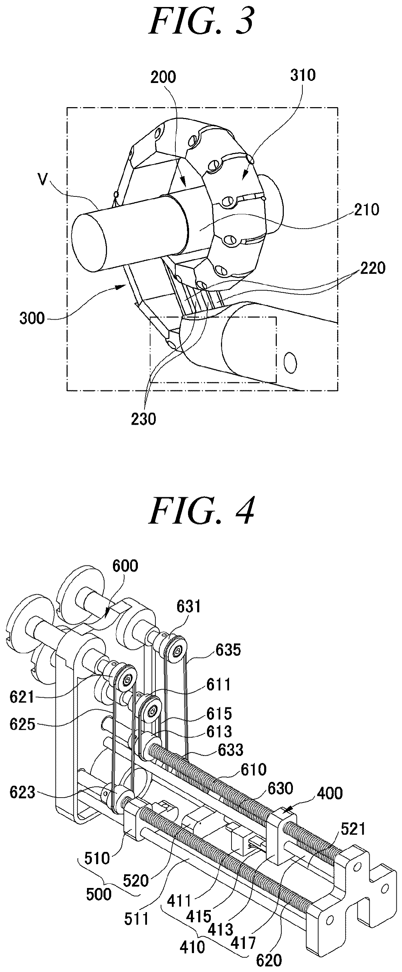

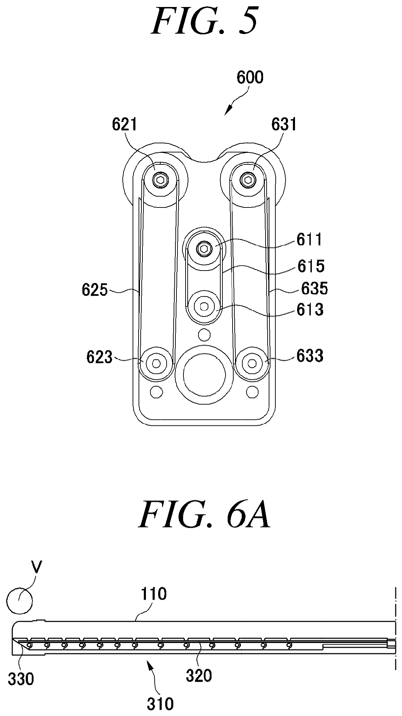

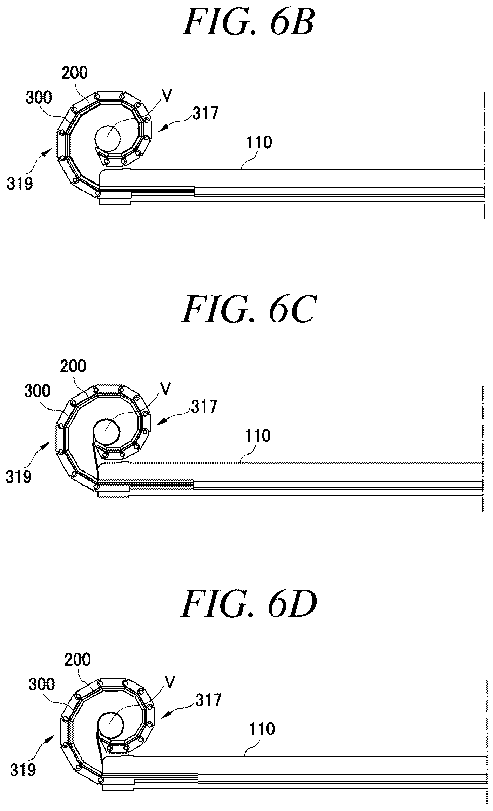

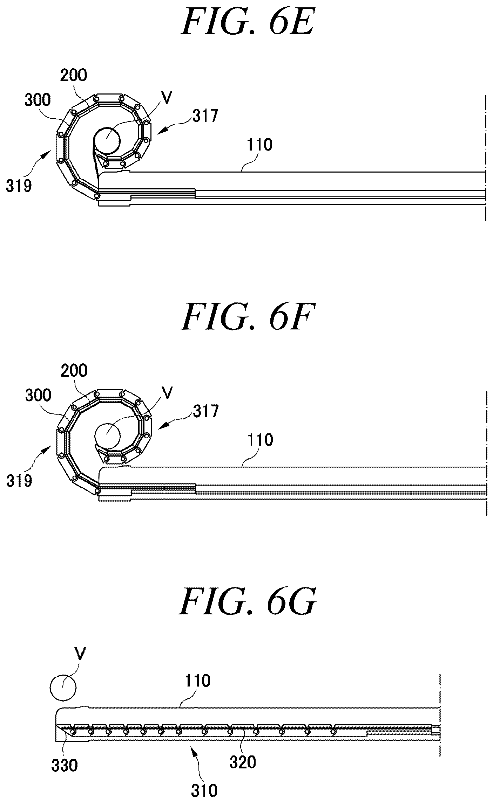

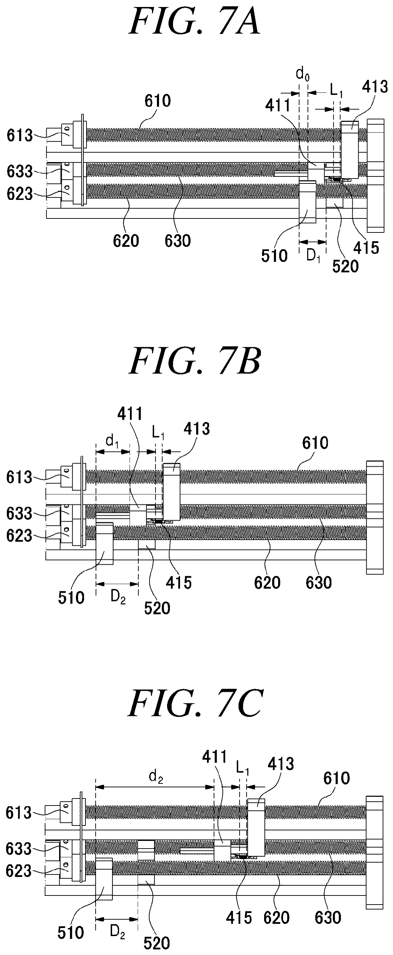

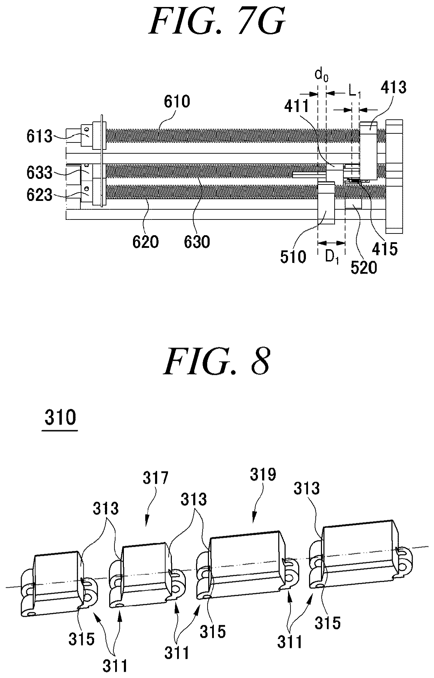

**FIG. 1** is a perspective view of an electrode apparatus 10 according to an embodiment of the present disclosure, **FIG. 2** is an inner perspective view of the electrode apparatus 10 according to an embodiment of the present disclosure, **FIG. 3** illustrates a state where an electrode guide 300 is located to guide an electrode unit 200 to enclose a tube, **FIG. 4** illustrates inner components of the electrode apparatus 10 according to an embodiment of the present disclosure, **FIG. 5** illustrates a motor interlocking unit 600 of the electrode apparatus 10 according to an embodiment of the present disclosure, **FIG. 6A** to **FIG. 6G** illustrate operation processes of the electrode guide according to an embodiment of the present disclosure, **FIG. 7A** to **FIG. 7G** illustrate operation processes of an electrode driving unit 400 and an electrode guide driving unit 500 according to an embodiment of the present disclosure, and **FIG. 8** is a perspective view illustrating a portion of joint units 310 of the electrode apparatus 10 according to an embodiment of the present disclosure.

Referring to **FIG. 1 to FIG. 4****,** the electrode apparatus 10 includes a main body 100, the electrode unit 200, the electrode guide 300, the electrode driving unit 400, the electrode guide driving unit 500, and the motor interlocking unit 600.

The main body 100 may include a shaft 110 extending in one direction.

The components for driving and controlling the electrode unit 200 and the electrode guide 300 may be located inside the main body 100. For example, the electrode driving unit 400 configured to drive and control the electrode unit 200 and the electrode guide driving unit 500 configured to drive and control the electrode guide 300 may be disposed inside the main body 100.

The electrode unit 200 is formed to be drawn out from one end of the shaft 110 and configured to denervate or modulate at least a part of nerves distributed on a tissue including a tube V in the body depending on manipulation by the operator. The electrode unit 200 is accommodated inside the shaft 110 and when the electrode apparatus operates, the electrode unit 200 can be drawn out by means of the electrode guide 300 which will be described later.

Referring to **FIG. 3****,** the electrode unit 200 may include a base unit 210, an energy transfer unit 220, and a sensor unit 230. In the electrode apparatus 10, an electrode encloses an outer surface of the tube or tube-shaped tissue V in the body and energy can be transferred through the energy transfer unit 220. To this end, the base unit 210 may be formed as a flexible printed circuit board.

The energy transfer unit 220 may be composed of two electrodes extending parallel to each other on the base unit 210. The base unit 210 and the energy transfer unit 220 may be configured to extend and enclose the tube V in the body or the like in a circumferential direction.

The energy transfer unit 220 may be made of a material, such as stainless steel or gold, which is harmless to the human body and conducts electricity well in order to block or denervate, or control or modulate the nerves.

Also, the energy transfer unit 220 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Further, the energy transfer unit 220 may be implemented as a flexible printed circuit board (PCB) for transferring RF energy, a transducer for transferring ultrasonic energy or a metal electrode for transferring high-voltage energy and thus may transfer energy to damage the nerves.

Furthermore, the sensor unit 230 may be formed on the base unit 210. For example, the sensor unit 230 may be a thermocouple that measures a temperature by contact with the tube V in the body or the like, and when neurotomy is performed with the electrode apparatus 10, the sensor unit 230 may monitor a temperature of a treatment site. Moreover, the sensor unit 230 may measure signals from the nerves on the tube.

The sensor unit 230 may be, for example, a thermocouple composed of a pair of copper and constantan.

The electrode guide 300 functions to bring the electrode unit 200 into contact with the tube V in the body.

The electrode guide 300 is coupled to the electrode unit 200 and guides the electrode unit 200 to be deformed into a wound state where the electrode unit 200 comes into contact with the tube V in the body.

Referring to **FIG. 3****,** and **FIG. 6A** to **FIG. 6G****,** the electrode guide 300 includes a plurality of joint units 310. The plurality of joint units 310 forms a curved winding path to enclose the circumference of the tube V in the body with the electrode unit 200 interposed therebetween. The state illustrated in **FIG.** 3 may be a state where the plurality of joint units 310 is completely drawn out and disposed along the curved winding path.

Referring to **FIG. 6A to FIG. 6G****,** the electrode guide 300 may further include a tip joint 330 and a wire 320. The tip joint 330 may support the electrode unit 200 and may be coupled to the end of the plurality of joint units 310 connected sequentially to each other.

The tip joint 330 may be drawn out from one end of the shaft 110 earlier than the plurality of joint units 310. For example, the tip joint 330 may be located close to the tube V in the body and may have a tapered shape that gradually decreases in width or thickness toward the end in order to suppress interference with the electrode unit 200 or maximize the surface enclosing the tube V in the body. The end of the electrode unit 200 may be fastened and fixed to the tip joint 330.

The wire 320 may be formed to sequentially penetrate the plurality of joint units 310.

Referring to **FIG. 8****,** each joint unit 310 may have a wire hole 315 in a longitudinal direction to allow penetration of the wire 320.

The end of the wire 320 sequentially penetrating the wire holes 315 may be coupled and fixed to the tip joint 330, and the wire 320 can slide with respect to each joint unit 310 in the wire hole 315 in the longitudinal direction.

Therefore, the wire 320 can guide the plurality of joint units 310 and the tip joint 330 to be located on the winding path and provide a force of pulling the plurality of joint units 310 and the tip joint 330 in a direction to be wound around the tube.

The wire 320 may be operated to protrude from one end of the shaft 110 together with the plurality of joint units 310. Here, the wire 320 may be designed to protrude less than the plurality of joint units 310 and thus can provide a force of pulling the plurality of joint units 310 along a curved path.

Each joint unit 310 may include hinge units 311 and winding support units 313. The hinge units 311 are configured for rotatable connection to adjacent joints and may be formed on one or both sides of the joint unit 310 in the longitudinal direction in which the joint units 310 are connected parallel to each other.

As illustrated in **FIG. 8****,** the hinge unit 311 may have a rotation axis in a direction intersecting the longitudinal direction so as to be connected to the hinge unit 311 of the adjacent joint unit 310. A hinge pin may be inserted into and fastened to each hinge unit 311 in the direction of the rotation axis.

The winding support units 313 are configured to support the plurality of joint units 310 on the winding path and may be formed on one or both sides of the joint unit 310 in the longitudinal direction to support the adjacent joint unit 310.

As illustrated in **FIG. 3** and **FIG. 8****,** the winding support unit 313 may be located adjacent to the hinge unit 311 in an inward direction of the electrode guide.

For example, the winding support unit 313 may be formed as a surface having a predetermined angle and area and supported by the adjacent winding support unit 313 in surface contact with each other, and, thus, a wound shape of the electrode guide 300 can be maintained.

The winding support unit 313 and the wire hole 315 may be formed at locations spaced apart from a rotation center of the hinge unit 311 in an inward direction toward the tube V in the body.

When the wire 320 is pulled backwards relative to the electrode guide 300 (when a length of the wire 320 drawn out from the shaft 110 is smaller than that of the joint units 310), a tensile force may be applied to the wire 320 in a direction of winding the electrode guide 300. On the other hand, the winding support units 313 may provide a force of supporting the joint units 310 to each other in a direction of suppressing winding of the electrode guide 300. Since the wire 320 and the winding support units 313 form a balanced force in opposite directions, the electrode guide 300 can be fixed on the winding path.

Further, the electrode guide 300 may include a first joint group 317 and a second joint group 319. That is, the plurality of joint units 310 may be divided into the first joint group 317 and the second joint group 319 having different lengths.

Due to a difference in length, the first joint group 317 may form a first radius of curvature and the second joint group 319 may form a second radius of curvature greater than the first radius of curvature. For example, the joint units (the first joint group) having a relatively small length may form a smaller radius of curvature and the joint units (the second joint group) having a relatively great length may form a greater radius of curvature.

When the joint units 310 located close to the tip joint 330 form a path having a smaller radius of curvature, a path along which the tip joint 330 enters a space between the tube V in the body and the shaft 110 may be formed. Also, the electrode guide 300 including the joint units 310 may have an overall spiral shape.

Referring to **FIG. 6A to FIG. 6G****,** the electrode guide 300 is accommodated together with the electrode unit 200 inside the shaft 110 and may protrude from one end in the forward direction while forming a curved winding path for surgical procedure.

For example, when the plurality of joint units 310 is sequentially drawn out, the plurality of joint units 310 may move along the curved winding path due to a difference in displacement from the wire 320 and thus may overall enclose the tube.

Further, the electrode guide 300 may be spaced apart from an outer circumferential surface of the tube and the electrode unit 200 located inside the wound electrode guide 300 may be in close contact with the outer circumferential surface of the tube.

The plurality of joint units 310 may be drawn out from the shaft 110 by means of the electrode guide driving unit 500 and wound in a direction to enclose the tube. Accordingly, a space where the electrode guide 300 operates can be minimized, and an operation of denervating or modulating nerves can be performed safely and accurately in a narrow space.

Meanwhile, the electrode unit 200 may be drawn out from the shaft 110 by means of the electrode driving unit 400 and may be wound in the direction to enclose the tube by means of the electrode guide 300. Specifically, when the electrode unit 200 moves together with the electrode guide 300 in the forward direction along the curved winding path and is completely drawn out from the shaft 110 and disposed, the electrode unit 200 may be gradually brought into close contact with the tube V in the body under the control of the electrode driving unit 400. Therefore, the electrode unit 200 is stably in close contact with the tube V in the body without damaging the tube V in the body, and, thus, an operation of denervating or modulating nerves can be performed.

Referring to **FIG. 4** and **FIG. 7A** **to** **FIG. 7G****,** the electrode driving unit 400 may be connected to the motor interlocking unit 600 configured to transfer a rotation amount generated by a plurality of motors and configured to move the electrode unit 200 in the forward and backward directions. In **FIG. 4****,** the electrode unit 200 and the electrode guide 300 are not illustrated.

The electrode driving unit 400 may include a tensile force maintenance unit 410 configured to provide a tensile force to the electrode unit 200, and the tensile force maintenance unit 410 may include an electrode connection unit 411, a first support frame 417, an electrode tensile force regulation block 413, a spring 415, and a force sensor (not shown).

As shown in **FIG. 4****,** one end of the electrode connection unit 411 may be connected to the electrode unit 200, and the other end of the electrode connection unit 411 may be connected to the electrode tensile force regulation block 413 and the spring 415. More specifically, a through-hole may be formed in the electrode connection unit 411 and one end of the electrode tensile force regulation block 413 may be inserted into the through-hole, and a first stop groove (not shown) may be formed in the electrode connection unit 411 and may protrude to the outside so as to be connected to one end of the spring 415.

The first support frame 417 may be provided to be fixed inside the main body 100, and the electrode tensile force regulation block 413 may be inserted into the first support frame 417 and may slide along an outer circumferential surface of the first support frame 417.

As the electrode tensile force regulation block 413 is inserted into the first support frame 417, it is possible to suppress deviation of the electrode tensile force regulation block 413 and fix a movement direction of the electrode tensile force regulation block 413.

The electrode tensile force regulation block 413 may be configured to move in the forward and backward directions through a first rotation shaft 610 coupled to the motor interlocking unit 600. Herein, the first rotation shaft 610 may be disposed at a location spaced apart from the first support frame 417 in a height direction so as to extend in the forward and backward directions, and the first rotation shaft 610 may have a thread thereon.

The electrode tensile force regulation block 413 may move in the forward and backward directions in engagement with the thread of the first rotation shaft 610.

One end of the electrode tensile force regulation block 413 may protrude to be inserted into the through-hole of the electrode connection unit 411. In this case, in order for the electrode tensile force regulation block 413 to provide a tensile force to the electrode unit 200, one end of the electrode tensile force regulation block 413 may protrude by a length enough not to deviate from the through-hole when a distance between the electrode tensile force regulation block 413 and the electrode connection unit 411 increases, and a tip end of the one end of the electrode tensile force regulation block 413 may further include a stopper configured to suppress deviation of the electrode connection unit 411.

The electrode tensile force regulation block 413 may include a second stop groove (not shown) at a location corresponding to the first stop groove so as to be connected to the other end of the spring 415.

The spring 415 may connect the electrode connection unit 411 and the electrode tensile force regulation block 413 and provide a tensile force to the electrode unit 200.

Specifically, the spring 415 may be connected to the first stop groove of the electrode connection unit 411 and the second stop groove of the electrode tensile force regulation block 413, and when the electrode tensile force regulation block 413 is moved in the backward direction through the first rotation shaft 610 coupled to the motor interlocking unit 600, the spring 415 may provide a tensile force to the electrode unit 200.

The force sensor may sense the tensile force generated in the spring 415. Specifically, when the electrode tensile force regulation block 413 is moved in the backward direction through the first rotation shaft 610 coupled to the motor interlocking unit 600, a tensile force is generated in the spring 415. In this case, the force sensor may sense the tensile force generated in the spring 415.

After the tensile force is generated, the force sensor may also sense a restoring force of the spring 415. That is, the force sensor may sense a tensile force or restoring force generated when the electrode tensile force regulation block 413 moves in the forward and backward directions.

Meanwhile, as shown in **FIG. 4****,** the electrode guide driving unit 500 may be connected to the electrode guide and configured to move the electrode guide in the forward and backward directions. Also, the electrode guide driving unit 500 may include a second support frame 511, a third support frame 521, a load block 510, and a wire block 520.

**The** second support frame 511 may be provided to be fixed inside the main body 100, and the load block 510 may be inserted into the second support frame 511 and may slide along an outer circumferential surface of the second support frame 511.

As the load block 510 is inserted into the second support frame 511, it is possible to suppress deviation of the load block 510 and fix a movement direction of the load block 510.

The third support frame 521 may be provided to be fixed inside the main body 100, and the wire block 520 may be inserted into the third support frame 521 and may slide along an outer circumferential surface of the third support frame 521.

As the wire block 520 is inserted into the third support frame 521, it is possible to suppress deviation of the wire block 520 and fix a movement direction of the wire block 520.

The load block 510 may be configured to move in the forward and backward directions through a second rotation shaft 620 connected to the joint units 310 and coupled to the motor interlocking unit 600. Herein, the second rotation shaft 620 may be disposed at a location spaced apart from the second support frame 511 in the height direction so as to extend in the forward and backward directions, and the second rotation shaft 620 may have a thread thereon.

The load block 510 may move in the forward and backward directions in engagement with the thread of the second rotation shaft 620.

The wire block 520 may be configured to move in the forward and backward directions through a third rotation shaft 630 connected to the wire 320 and coupled to the motor interlocking unit 600. Herein, the third rotation shaft 630 may be disposed at a location spaced apart from the third support frame 521 in the height direction so as to extend in the forward and backward directions, and the third rotation shaft 630 may have a thread thereon.

The wire block 520 may move in the forward and backward directions in engagement with the thread of the third rotation shaft 630.

Meanwhile, the motor interlocking unit 600 may be connected to the electrode driving unit 400 and the electrode guide driving unit 500 and may transfer a rotation amount generated by the plurality of motors to the electrode driving unit 400 and the electrode guide driving unit 500.

Herein, the plurality of motors may include a first motor configured to generate a rotation amount in order for the electrode tensile force regulation block 413 to move in the forward and backward directions, a second motor configured to generate a rotation amount in order for the load block 510 to move in the forward and backward directions, and a third motor configured to generate a rotation amount in order for the wire block 520 to move in the forward and backward directions.

For example, as shown in **FIG. 4** and **FIG. 5****,** the first motor (not shown) may be connected to a first motor pulley 611 formed in the motor interlocking unit 600, a first rotation shaft pulley 613 may be formed at one end of the first rotation shaft 610, and the first motor pulley 611 and the first rotation shaft pulley 613 may be connected by a first pulley connection member 615. Therefore, when the first motor (not shown) operates, torque of the first motor may be transferred to the first rotation shaft 610 through the first motor pulley 611, the first pulley connection member 615, and the first rotation shaft pulley 613, and, thus, the electrode tensile force regulation block may be moved in the forward and backward directions.

Also, the second motor (not shown) may be connected to a second motor pulley 621 formed in the motor interlocking unit 600, a second rotation shaft pulley 623 may be formed at one end of the second rotation shaft 620, and the second motor pulley 621 and the second rotation shaft pulley 623 may be connected by a second pulley connection member 625. Therefore, when the second motor (not shown) operates, torque of the second motor may be transferred to the second rotation shaft 620 through the second motor pulley 621, the second pulley connection member 625, and the second rotation shaft pulley 623, and, thus, the load block 510 may be moved in the forward and backward directions.

Further, the third motor (not shown) may be connected to a third motor pulley 631 formed in the motor interlocking unit 600, a third rotation shaft pulley 633 may be formed at one end of the third rotation shaft 630, and the third motor pulley 631 and the third rotation shaft pulley 633 may be connected by a third pulley connection member 635. Therefore, when the third motor (not shown) operates, torque of the third motor may be transferred to the third rotation shaft 630 through the third motor pulley 631, the third pulley connection member 635, and the third rotation shaft pulley 633, and, thus, the wire block 520 may be moved in the forward and backward directions.

As such, when the electrode tensile force regulation block 413, the load block 510, and the wire block 520 are moved in the forward and backward directions by the respective motors (not shown), the electrode unit 200 can be operated precisely when it is brought into close contact with an outer wall of a tube.

Also, the motor interlocking unit 600 operates in conjunction with a plurality of joint operating motors provided in the conventional surgical robot 1, and, thus, the electrode apparatus 10 can be used by being coupled to various surgical robots 1 without changes in structure.

Hereafter, driving of the electrode unit 200 and the electrode guide 300 by means of the electrode driving unit 400 and the electrode guide driving unit 500 will be described with reference to **FIG. 7A** to **FIG. 7G. FIG. 7A** to **FIG. 7G** illustrate states corresponding to the states illustrated in **FIG. 6A** to **FIG. 6G****.**

The electrode driving unit 400 and the electrode guide driving unit 500 illustrated in **FIG. 7A** may be in a state right before forward movement starts or right after backward movement ends. Therefore, as illustrated in **FIG. 6A****,** the electrode unit 200 and the electrode guide 300 may be in a state right before enclosing the circumference of the tube V in the body or right after the electrode unit 200 and the electrode guide 300 having enclosed the circumference of the tube V in the body are transitioned to the state before enclosing the tube V. That is, the electrode unit 200 and the electrode guide 300 may be in a state right before or right after neurotomy is performed with the electrode apparatus 10.

Herein, an initial distance between the load block 510 and the wire block 520 may be, for example, "D1", and an initial distance between the load block 510 and the electrode connection unit 411 may be, for example, "d0". Also, a length of the spring 415 may be, for example, "L1" in which the spring 415 does not change in length, and a force F measured by the force sensor may be "0".

Referring to **FIG. 7B****,** the electrode guide driving unit 500 may be connected to the motor interlocking unit 600 and may move in the forward direction through the motor, and when the electrode guide driving unit 500 moves in the forward direction, the electrode tensile force regulation block 413 may also move in the forward direction.

**As** illustrated in **FIG. 6B,** due to forward movement of the electrode guide driving unit 500 and the electrode tensile force regulation block 413, the electrode unit 200 and the electrode guide 300 may be drawn out from the shaft 110 in the forward direction and wound to enclose the circumference of the tube V in the body.

Specifically, the electrode tensile force regulation block 413 may move in the forward direction through the first rotation shaft 610 connected to the first motor (not shown) along a path provided by the first support frame 417, the load block 510 may move in the forward direction through the second rotation shaft 620 connected to the second motor along a path provided by the second support frame 511, and the wire block 520 may move in the forward direction through the third rotation shaft 630 connected to the third motor along a path provided by the third support frame 521.

Accordingly, the electrode guide 300 connected to the electrode guide driving unit 500 may be drawn out from the shaft 110 in the forward direction, and the electrode unit 200 of which one end is connected to the electrode connection unit 411 may be drawn out from the shaft 110.

When the electrode guide driving unit 500 moves in the forward direction, a first rotation speed of the second rotation shaft 620 may be set to be higher than a second rotation speed of the third rotation shaft 630, and, thus, the load block 510 may move further in the forward direction than the wire block 520.

For example, as shown in **FIG. 6B,** a rotation amount of the second motor may be set to be greater than that of the third motor, and, thus, the distance between the load block 510 that moves in the forward direction by a first rotation speed of the second rotation shaft 620 connected to the second motor (not shown) and the wire block 520 that moves in the forward direction by a second rotation speed of the third rotation shaft 630 connected to the third motor may be increased to "D2".

Also, a third rotation speed of the first rotation shaft 610 may be set to be lower than the second rotation speed of the third rotation shaft 630, and, thus, when the electrode driving unit 400 and the electrode guide driving unit 500 move in the forward direction, the electrode tensile force regulation block 413, the load block 510, and the wire block 520 may move in the forward direction by different lengths, respectively.

For example, as shown in **FIG. 6A** and **FIG. 6B,** the rotation amount of the third motor may be set to be greater than that of the first motor, and as described above, the first rotation speed of the second rotation shaft 620 may be set to be higher than the second rotation speed of the third rotation shaft 630, and, thus, the distance between the load block 510 that moves in the forward direction by the first rotation speed of the second rotation shaft 620 connected to the second motor (not shown) and the electrode connection unit 411 connected through the spring 415 to the electrode tensile force regulation block 413 that moves in the forward direction by the third rotation speed of the first rotation shaft 610 connected to the first motor may be increased to "d1".

Further, the length of the spring 415 may be, for example, "L1" in which the spring 415 does not change in length, and the force F measured by the force sensor (not shown) may be "0".

Referring to **FIG. 7C****,** the electrode guide driving unit 500 may be kept in a stationary state and the electrode tensile force regulation block 413 may be moved in the backward direction through the first rotation shaft 610 connected to the first motor.

Herein, the distance between the load block 510 and the wire block 520 may be "D2" because they are kept in a stationary state at a location to which they have moved in the forward direction. Also, the load block 510 is kept in a stationary state and the electrode tensile force regulation block 413 is moved in the forward direction through the first rotation shaft 610 connected to the first motor, and, thus, the distance between the load block 510 and the electrode connection unit 411 connected to the electrode tensile force regulation block 413 through the spring 415 may be increased to "d2". Herein, the increased distance "d2" may be in inverse proportion to the diameter of the tube in contact with the electrode unit 200.

Also, a length of the spring 415 may be "L1" in which the spring 415 does not change in length. Further, a force F measured by the force sensor may be "0".

Due to the increased length "d2", the electrode unit 200 may be brought into contact with the tube V in the body without a change in length of the spring 415 as illustrated in **FIG. 6C****.**

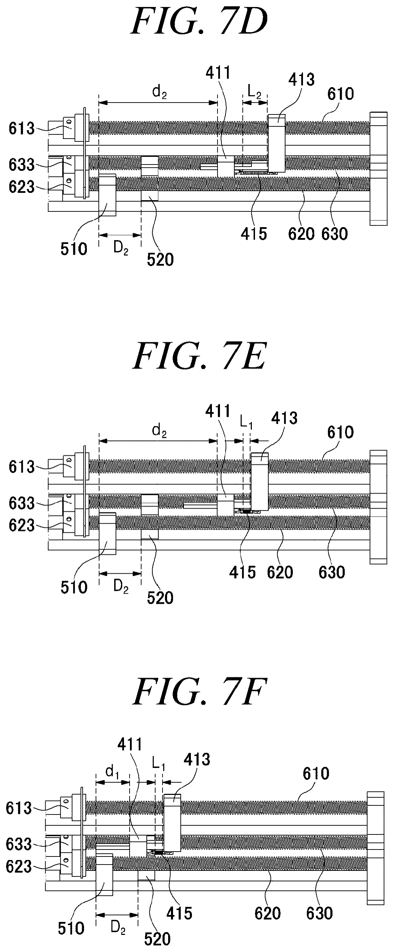

Referring to **FIG. 7D****,** the electrode guide driving unit 500 may be kept in a stationary state and the electrode tensile force regulation block 413 may be further moved in the backward direction through the first rotation shaft 610 connected to the first motor, and, thus, the length of the spring 415 may be increased.

Specifically, the distance between the load block 510 and the wire block 520 may be "D2" because they are kept in a stationary state at a location to which they have moved in the forward direction. Also, the electrode connection unit 411 is connected to the electrode unit 200 of which one end is in contact with the tube V in the body and kept in a stationary state, and, thus, the distance between the load block 510 and the electrode connection unit 411 may be "d2".

Further, the electrode tensile force regulation block 413 may be further moved in the backward direction through the first rotation shaft 610 connected to the first motor, and, thus, the length of the spring 415 connecting the electrode connection unit 411 and the electrode tensile force regulation block 413 may be increased.

The length of the spring 415 may be, for example, "L2" in which the spring 415 increases in length, and a force measured by the force sensor may be generated.

The force measured by the force sensor may be a tensile force generated when the length of the spring 415 increases by a predetermined length.

**As** such, the electrode guide driving unit 500 is kept in a stationary state and the electrode tensile force regulation block 413 is further moved in the backward direction, and, thus, the spring 415 increases in length. The electrode unit 200 brought into close contact with the tube V in the body with a predetermined tensile force may transfer energy for damaging nerves to enable neurotomy.

Then, referring to **FIG. 7E****,** the electrode guide driving unit 500 is kept in a stationary state and the electrode tensile force regulation block 413 is moved in the forward direction through the first rotation shaft 610 connected to the first motor by an amount equivalent to the amount of backward movement of the electrode tensile force regulation block 413 as shown in FIG. 7D. Thus, the spring 415 may return back to its original state.

Specifically, the distance between the load block 510 and the wire block 520 may be "D2" because they are kept in a stationary state at a location to which they have moved in the forward direction, and the electrode connection unit 411 may be kept in a stationary state. Thus, the distance between the load block 510 and the electrode connection unit 411 may be "d2". Further, the electrode tensile force regulation block 413 is moved in the forward direction through the first rotation shaft 610 connected to the first motor by an amount equivalent to the amount of backward movement as described above, and, thus, the length of the spring 415 may be decreased to a predetermined length.

Accordingly, all the tensile force generated in the spring 415 is removed, and, thus, the electrode unit 200 may be brought into contact with the tube V in the body without a change in length of the spring 415 as illustrated in **FIG. 6E****.** Therefore, the length of the spring 415 may be "L1" in which the spring 415 does not change in length, and the force F measured by the force sensor may be "0".

Referring to **FIG. 7F****,** the electrode guide driving unit 500 may be kept in a stationary state, and the electrode tensile force regulation block 413 may be moved in the forward direction through the first rotation shaft 610 connected to the first motor by an amount equivalent to the amount of backward movement as shown in **FIG. 7C****.**

Specifically, the distance between the load block 510 and the wire block 520 may be "D2" because they are kept in a stationary state at a location to which they have moved in the forward direction, and the distance between the load block 510 and the electrode connection unit 411 connected to the electrode tensile force regulation block 413 through the spring 415 may return to "d1". The length of the spring 415 may be "L1" in which the spring 415 does not change in length, and the force F measured by the force sensor may be "0".

Also, the electrode unit 200 which is spaced apart from the electrode guide 300 and in close contact with the tube V in the body may be attached again to the electrode guide 300.

When backward movement of the electrode driving unit 400 and the electrode guide driving unit 500 is completed, the distance between the load block 510 and the wire block 520 may be "D1" and the distance between the load block 510 and the electrode connection unit 411 may be "d0" as illustrated in **FIG. 7G****.** Also, the length of the spring 415 may be "L1" in which the spring 415 does not change in length, and the force F measured by the force sensor may be "0".

Herein, **FIG. 7A** and **FIG. 7G** illustrate the same state right before forward movement of the electrode driving unit 400 and the electrode guide driving unit 500 starts or right after backward movement ends. Therefore, as illustrated in **FIG. 6G****,** the electrode unit 200 and the electrode guide 300 may be in a state right before enclosing the circumference of the tube V in the body or right after the electrode unit 200 and the electrode guide 300 having enclosed the circumference of the tube V in the body are transitioned to the state before enclosing the tube V. That is, the electrode unit 200 and the electrode guide 300 may be in a state right before or right after neurotomy is performed with the electrode apparatus 10.

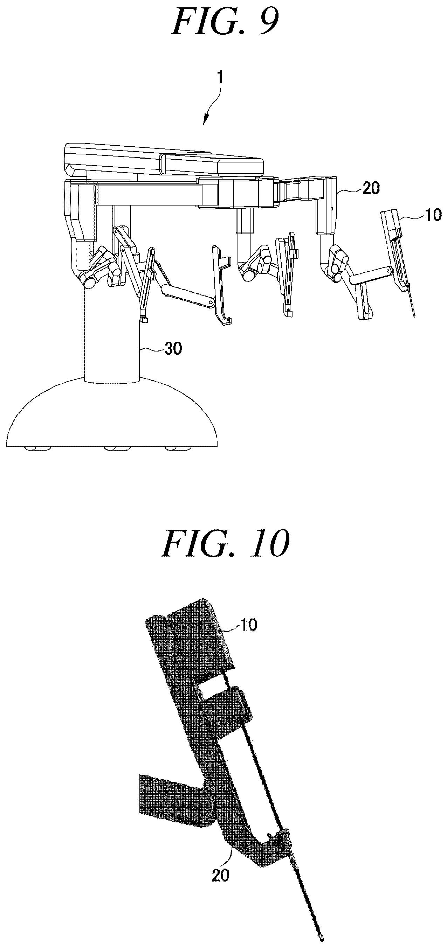

**FIG. 9** is a perspective view of a surgical robot 1 using the electrode apparatus 10 according to another embodiment of the present disclosure, and **FIG. 10** illustrates the electrode apparatus 10 coupled to a robot arm 20 of the surgical robot 1 according to another embodiment of the present disclosure.

As illustrated in **FIG. 9** and **FIG. 10****,** the surgical robot 1 using the electrode apparatus 10 according to an embodiment of the present disclosure may include the electrode apparatus 10, the robot arm 20, and a main body 30 configured to support the robot arm 20.

The robot arm 20 may include a plurality of motors configured to transfer a rotation amount to the electrode driving unit 400 and the electrode guide driving unit 500 through the motor interlocking unit 600.

The plurality of motors may include a first motor (not shown) configured to generate a rotation amount in order for the electrode tensile force regulation block 413 to move in the forward and backward directions, a second motor (not shown) configured to generate a rotation amount in order for the load block 510 to move in the forward and backward directions, and a third motor (not shown) configured to generate a rotation amount in order for the wire block 520 to move in the forward and backward directions.

The surgical robot 1 may further include a controller.

The controller may include a display configured to display an image of a surgical site, a first joystick configured to control an operation of the robot arm 20, a pair of grip portions configured to control an operation of the electrode apparatus 10, and a pair of pedals configured to control an operation of the electrode apparatus 10.

However, the controller is not limited thereto and can have various modified configurations as long as it can operate the robot arm 20 and the electrode apparatus 10 according to the user's intention.

As described above, in the electrode apparatus 10 according to an embodiment of the present disclosure, the electrode guide 300 is located to be in close contact with the tube V in the body and gradually brings the electrode unit 200 into close contact with the outer wall of the tube. Thus, it is possible to safely bring a component including an electrode into close contact with the outer wall of the tube without damaging the tube V in the body which can be easily damaged by external stimuli.

Also, in the electrode apparatus 10, the motor interlocking unit 600 transfers a rotation amount to each of the electrode driving unit 400 and the electrode guide driving unit 500 through a plurality of motors (not shown). Thus, it is possible to regulate the electrode unit 200 to operate precisely when the electrode unit 200 is brought into close contact with the outer wall of the tube. Also, the electrode apparatus 10 can be used by being coupled to a joint operating motor provided in various surgical robots 1 without changes in structure.

The above description of the present disclosure is provided for the purpose of illustration, and it would be understood by a person with ordinary skill in the art to which the present disclosure belongs that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner, likewise, components described to be distributed can be implemented in a combined manner.

Further, the scope of the present disclosure is defined by the following claims rather than by the detailed description of the embodiment, and it should be understood that all modifications and embodiments conceived from the meaning and scope of the claims and their equivalents are included in the scope of the present disclosure.

## Claims

1. An electrode apparatus for blocking or controlling nerves in a body, comprising:
a main body including a shaft;
an electrode unit formed to be drawn out from one end of the shaft and configured to denervate or modulate at least a part of nerves on a tube in the body;
an electrode guide coupled to at least a part of the electrode unit and to guide the electrode unit to be brought into contact with the tube in the body;
an electrode driving unit including a tensile force maintenance unit connected to the electrode unit and configured to move the electrode unit in forward and backward directions and provide a tensile force to the electrode unit;
an electrode guide driving unit connected to the electrode guide and configured to move the electrode guide in the forward and backward directions; and
a motor interlocking unit connected to the electrode driving unit and the electrode guide driving unit and configured to transfer a rotation amount generated by a plurality of motors to the electrode driving unit and the electrode guide driving unit.

2. The electrode apparatus of Claim 1,
wherein the tensile force maintenance unit includes:
an electrode connection unit connected to one end of the electrode unit;
an electrode tensile force regulation block configured to move in the forward and backward directions through a first rotation shaft coupled to the motor interlocking unit; and
a spring configured to connect the electrode connection unit to the electrode tensile force regulation block and provide a tensile force to the electrode unit.

3. The electrode apparatus of Claim 2,
wherein the electrode guide includes a plurality of joint units and a wire connecting the plurality of joint units to each other, and
the electrode guide driving unit includes:
a load block configured to move in the forward and backward directions through a second rotation shaft connected to the joint units and coupled to the motor interlocking unit; and
a wire block configured to move in the forward and backward directions through a third rotation shaft connected to the wire and coupled to the motor interlocking unit.

4. The electrode apparatus of Claim 3,
wherein when the electrode guide driving unit moves in the forward and backward directions, a first rotation speed of the second rotation shaft is set to be higher than a second rotation speed of the third rotation shaft and the load block moves further in the forward and backward directions than the wire block.

5. The electrode apparatus of Claim 3,
wherein when the electrode guide driving unit moves in the forward and backward directions, the electrode tensile force regulation block moves together in the forward and backward directions.

6. The electrode apparatus of Claim 5,
wherein a third rotation speed of the first rotation shaft is set to be lower than a first rotation speed of the second rotation shaft.

7. The electrode apparatus of Claim 2,
wherein after the electrode guide driving unit moves in the forward direction and stops, the electrode tensile force regulation block moves in the backward direction.

8. The electrode apparatus of Claim 7,
wherein after a predetermined tensile force is generated in the electrode unit as the electrode tensile force regulation block moves in the backward direction, the electrode tensile force regulation block stops.

9. A surgical robot using an electrode apparatus for blocking or controlling nerves in a body, comprising:
the electrode apparatus of Claim 1;
a robot arm including a plurality of motors connected to the motor interlocking unit and configured to transfer a rotation amount to the electrode driving unit and the electrode guide driving unit through the motor interlocking unit; and
a main body configured to support the robot arm.

10. The surgical robot of Claim 9,
wherein the plurality of motors includes:
a first motor configured to generate a rotation amount in order for the electrode tensile force regulation block to move in the forward and backward directions;
a second motor configured to generate a rotation amount in order for the load block to move in the forward and backward directions; and
a third motor configured to generate a rotation amount in order for the wire block to move in the forward and backward directions.
